# EUROPEAN PATENT APPLICATION

(11) **EP 4 825 403 A1**
(43) Date of publication of application: **21.10.2026**
(21) Application number: 25766464.9
(22) Date of filing: 26.03.2025
(51) Int. Cl.: A61L 31/06, A61L 31/14

(54) **SELF-ADHESIVE MATERIAL, AND PREPARATION METHOD THEREFOR AND USE THEREOF**

(30) Priority: 21.02.2025 CN 202510194327
(71) Applicant: Westlake University, Hangzhou, Zhejiang 310024 (CN); Shanghai Sixth People's Hospital, Shanghai 200233 (CN)
(72) Inventor: YANG, Jian, Hangzhou, Zhejiang 310024 (CN); ZHANG, Lujiao, Hangzhou, Zhejiang 310024 (CN); LIU, Shen, Hangzhou, Zhejiang 310024 (CN); HAN, Changhao, Hangzhou, Zhejiang 310024 (CN)
(74) Representative: WSL Patentanwälte Partnerschaft mbB
(86) International application number: PCT/CN2025/085058
(87) International publication number: WO 2026/174637

(57) **Abstract**

The present application relates to the field of biomedical materials, specially relates to self-adhesive material, preparation method and uses thereof. A provided method for preparing a self-adhesive material includes: reacting component A and component B to form a citric acid-based prepolymer; the component A is at least one compound containing at least two functional groups which are capable of reaction and at terminal groups or on side chains of the component A, the functional groups are the same or different; the functional group is selected from at least one of hydroxyl, amino, sulfydryl, epoxy, carboxyl, acid anhydride, and organic substituents; the component B is citric acid and/or derivatives thereof; the reaction comprises a polycondensation reaction, a substitution reaction, or a transesterification reaction; performing a cross-linking reaction of the citric acid-based prepolymer and isocyanate via a urethane bond or an amide bond; the material obtained by the above preparation method has good self-adhesive properties between the contact surfaces, which can be fixed without suture, thereby greatly reducing the difficulty of surgical operation and the risk of infection, reducing tissue injury and improving the success rate of surgery.

## Description

### CROSS REFERENCE TO RELATED APPLICATION

The present application claims priority to Chinese Patent Application No. 202510194327.5, filed to the China National Intellectual Property Administration on February 21, 2025, entitled "SELF-ADHESIVE MATERIAL, PREPARATION METHOD AND USE THEREOF", and the entire contents of which are incorporated herein by reference.

### TECHNICAL FIELD

The present application relates to the field of biomedical materials, and in particular to a self-adhesive material, a preparation method and use thereof.

### BACKGROUND

Biomembranes in the field of biomedical materials are generally used as materials for replacing and repairing human tissues. These materials are applied to variety application scenarios, such as wound dressings, medical adhesives, anti-adhesion membranes, etc. Among them, the function of anti-adhesion membrane is to prevent tissue adhesion. Tissue adhesion refers to a pathological condition in which the contact surfaces of adjacent tissues or organs are connected by cellulose or newly formed fibrous tissue in scar tissue. It is a common clinical phenomenon after surgery and an essential process for patients to heal. A dhesion formation is typically associated with surgery, trauma, inflammation, infection, intraperitoneal foreign bodies, or other predisposing factors. For example, tendon adhesion is closely related to tendon healing process and can be divided into two mechanisms: endogenous healing and exogenous healing. Endogenous healing achieves tendon healing through the proliferation of tendon surface fibroblasts themselves, which can prevent tendon adhesion. However, exogenous healing involves the formation of new granulation tissue on the tendon rupture face by the subcutaneous tissue and synovium of the tendon, thereby resulting in the formation of scar tissue, leading to adhesion. In addition, the disruption of tendon blood supply and integrity, as well as inflammatory reactions in the peritendinous tissues, are also important causes of tendon adhesion. Moreover, adhesions may cause various complications such as small intestinal obstruction, secondary infertility, pain in the morbid site or joint movement dysfunction. Due to the serious complications that adhesions may cause, preventing the occurrence of adhesion is of great significance for improving patients' quality of life and reducing medical costs.

At present, the anti-adhesion biomembrane material used as a physical barrier can inhibit the adhesion formation by isolating the injury site from surrounding tissues, which has become a commonly used method in clinical practice. Currently, researchers have developed anti-adhesion membranes prepared from various biomaterials, including natural materials (such as collagen and hyaluronic acid), synthetic materials (such as polylactic acid and polyglycolic acid) and composite materials. These materials have improved the effectiveness of tissue repair to a certain extent. For example, it is reported in patent document CN116077739A that a decellularized pericardium with a double-sided structure can effectively block adhesions. A composite nanofiber membrane is proposed in CN111714696A that combines good biocompatibility and mechanical properties.

However, there are still some shortcomings in the existing anti-adhesion biomembrane materials: most anti-adhesion biomembranes require suturing for fixation, which increases the difficulty of surgical operations and the risk of infection. In addition, the degradation rate, mechanical strength and tissue biocompatibility of the material still need further optimization. Therefore, developing a self-adhesive, well biocompatible, and absorbable anti-adhesion biomaterial holds great significance for improving the effectiveness of tissue repair.

### SUMMARY OF THE INVENTION

Therefore, the technical problem to be solved in the present application is to overcome the defects of anti-adhesive biomembrane material in prior art, which require suture fixation, thereby increasing the difficulty of surgical operation and the risk of infection, and have relatively unsatisfactory degradation rate, mechanical strength and tissue biocompatibility. Therefore, a self-adhesive material, a preparation method and use thereof are provided. The self-adhesive material can be suture-free due to its self-adhesive property, thereby reducing the difficulty of surgical operation and the risk of infection when applied to anti-adhesion membranes. Furthermore, the self-adhesive material is degradable and capable of being completely degraded and absorbed in the body of humans or non-human animals. The self-adhesive material exhibits good biocompatibility and adjustable mechanical properties. Meanwhile, this self-adhesive material can be endowed with multiple functions such as luminescence localization, drug delivery, repair promotion, antibacterial and angiogenesis promotion.

For the above object, the present application provides the following technical solutions:
The present application provides a method for preparing a self-adhesive material, the method comprises:
reacting component A and component B to form a citric acid-based prepolymer; wherein the component A is at least one compound containing at least two functional groups which are capable of reaction and are at terminal groups or on side chains of the component A, and the functional groups are the same or different; the functional group is selected from at least one of hydroxyl, amino, sulfydryl, epoxy, carboxyl, acid anhydride, and organic substituents; the component B is citric acid and/or derivatives thereof; the reaction comprises a polycondensation reaction, a substitution reaction, or a transesterification reaction; and
performing a cross-linking reaction of the citric acid-based prepolymer and isocyanate via a urethane bond or an amide bond.

Optionally, the isocyanate is selected from at least one of 1,6-hexamethylene diisocyanate, isophorone diisocyanate, toluene diisocyanate, lysine diisocyanate, naphthalene diisocyanate, 1,4-cyclohexane diisocyanate, 4,4'-diphenylmethane diisocyanate and 4,4'-methylenebis(cyclohexyl isocyanate).

Optionally, the isocyanate is selected from at least one of 1,6-hexamethylene diisocyanate, lysine diisocyanate and isophorone diisocyanate.

Optionally, when the citric acid-based prepolymer is in a solid state, before conducting the cross-linking reaction of the urethane bond or the amide bond, the citric acid-based prepolymer is dissolved in an organic solvent to obtain a citric acid-based prepolymer solution; the isocyanate is added to the citric acid-based prepolymer solution to obtain a mixed solution, and then the cross-linking reaction of the urethane bond or the amide bond is performed;
a mass percentage of the citric acid-based prepolymer in the citric acid-based prepolymer solution is in a range from 30% to 60%; and
the amount of the isocyanate added is greater than 0% and less than or equal to 50% by mass percentage of the citric acid-based prepolymer.

Optionally, the cross-linking reaction of the urethane bond or the amide bond is carried out at room temperature for a duration ranging from 0 h to 24 h, with the duration being greater than 0 h to ensure that the added organic solvent is completely evaporated.

Optionally, a step of preparing the self-adhesive material into a membrane, comprising spin coating, solution casting, or solution casting to form a membrane from a mixed solution of the citric acid-based prepolymer and the isocyanate during the cross-linking reaction of the urethane bond or the amide bond.

Optionally, the spin coating is performed at a rotation speed ranging from 1000 rpm to 3000 rpm and for a duration ranging from 0 min to 3 min, with the duration being greater than 0 min.

Optionally, the thickness of the membrane is in a range from 10 µm to 500 µm. Thinner membrane materials can better adhere to the tissue injury site, reduce foreign body sensation, and maintain a self-adhesive state effectively due to the small tensile force subjected.

Optionally, the component A is a hydrophobic compound or a hydrophilic compound;
and/or, when the component A is a hydroxy compound, including but not limited to ethylene glycol, hexanediol, octanediol, polyethylene glycol, bis(4-hydroxyphenyl) disulfide, resveratrol, bis(2-hydroxyethyl) disulfide, isosorbide, xylitol, hydroxyacetate or N-methyldiethanolamine;
and/or, when the component A is an amino acid or other compounds containing the amino group or the sulfydryl group, including but not limited to serine, phosphoserine, cysteine, glutamine or polyaniline;
and/or, when the component A is an epoxy compound, including but not limited to epoxidized soybean oil, epoxy resin, epichlorohydrin or lactide;
and/or, when the component A is a compound containing the carboxyl group or the acid anhydride, including but not limited to folic acid, tartaric acid, adipic acid, succinic acid, fumaric acid, salicylic acid, acetic anhydride or maleic anhydride;
and/or, when the component A is a compound containing the organic substituents, including but not limited to polyphosphazene;
and/or, the citric acid and/or derivatives thereof include but are not limited to citric acid, trimethyl citrate, triethyl citrate, tributyl citrate, acetyl triethyl citrate, stearyl monoglyceride citrate or citric acid luminescent molecules.

The present application provides a self-adhesive material prepared by the described method for preparing a self-adhesive material.

The present application provides the use of the self-adhesive material described in any of the following:
the use of preparing a suture-free and/or an anti-adhesion biomembrane; the use of the suture-free and/or the anti-adhesion biomembrane as a tissue anti-adhesion membrane for tendons, abdominal cavity, pelvic cavity, nerves, and spinal site.

The technical solution of the present application has the following advantages:
1. The present application provides a method for preparing a self-adhesive material, which comprises: reacting component A and component B to form a citric acid-based prepolymer; the component A is at least one compound containing at least two functional groups which are capable of reaction and are at terminal groups or on side chains of the component A, wherein the functional groups are the same or different; the functional group is selected from at least one of hydroxyl, amino, sulfydryl, epoxy, carboxyl, acid anhydride, and organic substituents; the component B is citric acid and/or derivatives thereof; the reaction comprises a polycondensation reaction, a substitution reaction, or a transesterification reaction; and performing a cross-linking reaction of the citric acid-based prepolymer and isocyanate via a urethane bond or an amide bond. The research in the present application has found that, by directly crosslinking the citric acid-based prepolymer with isocyanate, the crosslinking points are urethane bonds or amide bonds, such as the reaction of isocyanate group with hydroxyl group to form a urethane bond crosslink. The urethane bonds or amide bonds formed by crosslinking enhance the hydrogen bonding interaction between the contact surfaces of the material, resulting in good self-adhesion between the contact surfaces of the obtained material. This allows for suture-free fixation during application, greatly reducing the difficulty of surgical operation and the risk of infection. The obtained material can be directly attached to the surface of the defect site, effectively avoiding the drawbacks of traditional membranes that require suturing and the potential inflammation caused by suture wear on tissues. This greatly simplifies surgical operation, reduces tissue injury and improves the success rate of surgery.

Furthermore, the self-adhesive material also exhibits satisfactory degradation rate, mechanical strength and histocompatibility. The prepared citric acid-based material can be completely degraded in vivo, and the exogenous citric acid released through degradation can promote the repair of the defect site through metabolic regulation.

In summary, compared with anti-adhesion membranes prepared from traditional synthetic materials, the citric acid-based material prepared in the present application has low production costs, simple preparation methods, better biocompatibility, stronger self-adhesion, simpler operation methods and potential multifunctionality (such as antibacterial, in vivo imaging and repair promotion). This material holds broad transformation and application prospects in the field of tissue defect repair, and is effective in preventing adhesion, promoting tissue regeneration and improving postoperative function of patients.

2. The present application provides a method for preparing a self-adhesive material, which comprises that, the isocyanate is selected from at least one of 1,6-hexamethylene diisocyanate, isophorone diisocyanate, toluene diisocyanate, lysine diisocyanate, naphthalene diisocyanate, 1,4-cyclohexane diisocyanate, 4,4'-diphenylmethane diisocyanate and 4,4'-methylenebis(cyclohexyl isocyanate). By directly crosslinking the citric acid-based prepolymer with isocyanate, the urethane bond or amide bond formed by crosslinking enhances the hydrogen bonding interaction between the contact surfaces of the material, resulting in good self-adhesion between the contact surfaces of the obtained material. This allows for suture-free fixation during application, greatly reducing the difficulty of surgical operation and the risk of infection. The aforementioned isocyanates can all achieve this function by adjusting the addition ratio with the prepolymer. Furthermore, since the self-adhesive material need to possess a windable performance, being too hard or too brittle will affect their winding performance. Therefore, if the molecular structure of the selected isocyanate is too rigid or the amount used is excessive, it will lead to an increase in material strength, which is not conducive to winding. When implanted into the body, it is prone to cause a significant foreign body sensation and easily trigger inflammatory reactions. However, the molecular structure of the aforementioned isocyanate ensures that the prepared self-adhesive material possesses a windable performance. Furthermore, 1,6-hexamethylene diisocyanate, lysine diisocyanate and isophorone diisocyanate belong to isocyanates with relatively flexible molecular chain structures, and the prepared self-adhesive material has excellent windable performance.

3. The present application provides a method for preparing a self-adhesive material, which comprises: when the citric acid-based prepolymer is in a solid state, before conducting the cross-linking reaction of the urethane bond or the amide bond, the citric acid-based prepolymer is dissolved in an organic solvent to obtain a citric acid-based prepolymer solution; the isocyanate is added to the citric acid-based prepolymer solution to obtain a mixed solution, and then the cross-linking reaction of the urethane bond or the amide bond is performed; the mass percentage of the citric acid-based prepolymer in the citric acid-based prepolymer solution is in a range from 30% to 60%; the amount of the isocyanate added is greater than 0% and less than or equal to 50% by mass percentage of the citric acid-based prepolymer. By controlling the ratio of the citric acid-based prepolymer to isocyanate, the adhesion of the material can be improved. The reason is that the urethane bond or amide bond formed by the direct crosslinking of isocyanate with the citric acid-based prepolymer enhances the hydrogen bonding interaction between the contact surfaces of the material, resulting in good self-adhesion between the contact surfaces of the obtained material. Therefore, the ratio of the citric acid-based prepolymer to isocyanate will directly affects the content of hydrogen bonds, thereby affecting the adhesive performance of the material. Furthermore, since the self-adhesive material need to possess a windable performance, being too hard or too brittle will affect their winding performance. Therefore, if the amount of the selected isocyanate used is excessive, it will lead to an increase in material strength, which is not conducive to winding. When implanted into the body, it is prone to cause a significant foreign body sensation and easily trigger inflammatory reactions. By selecting the above-mentioned ratio, a self-adhesive material with superior windable performance can be prepared.

4. The present application provides a method for preparing a self-adhesive material. A step of preparing the self-adhesive material into a membrane, comprises spin coating a mixed solution of the citric acid-based prepolymer and the isocyanate into a membrane during the cross-linking reaction of the urethane bond or the amide bond. The spin coating is performed at a rotation speed ranging from 1000 rpm to 3000 rpm and for a duration ranging from 0 min to 3 min, with the duration being greater than 0 min. By adopting the method of spin coating to prepare the membrane, the solvent evaporates rapidly during rapid rotation process, thereby ensuring that the thickness of the prepared membrane is controllable and dries quickly.

5. The present application provides a method for preparing a self-adhesive material, wherein the component A is a hydrophobic compound or a hydrophilic compound. The present application involves the preparation of hydrophilic or hydrophobic materials by altering the structure of the component A. In the present application, a hydrophobic anti-adhesion biomembrane can be obtained when selecting such as octanediol compounds, and a hydrophilic anti-adhesion biomembrane can be obtained when selecting such as polyethylene glycol-based hydrophilic compounds.

### DESCRIPTION OF THE DRAWINGS

To more clearly illustrate the specific embodiments of the present application or the technical solutions in the prior art, the accompanying drawings required for the description of the specific embodiments or prior art will be briefly introduced below. It is apparent that the accompanying drawings described below are some embodiments of the present application. For those ordinary skilled in the art, other drawings can be obtained based on these accompanying drawings without creative labor.
FIG. 1 is a real-life rendering of the suture-free anti-adhesion membrane prepared according to Example 1.
FIG. 2 is a scanning electron microscopy image of the surface and cross-section of the suture-free anti-adhesion membrane prepared according to Example 1.
FIG. 3 is an infrared spectrum of the suture-free anti-adhesion membrane prepared according to Example 1.
FIG. 4 is a self-adhesive effect image of the suture-free anti-adhesion membrane prepared according to Example 1.
FIG. 5 is a statistical graph showing the citric acid content released from the suture-free anti-adhesion membrane prepared from the prepolymer according to Example 1 using different mass ratios of isocyanate, as well as the intracellular citric acid content after co-incubation. The left side shows the citric acid content released from the suture-free anti-adhesion membrane into the supernatant; the right side shows the citric acid content in the cells after co-incubation of the supernatant with the cells.
FIG. 6 is a water contact angle image of the suture-free anti-adhesion membrane prepared according to Example 2.
FIG. 7 is a self-adhesive effect image of the suture-free anti-adhesion membrane prepared according to Example 2.
FIG. 8 is a fluorescence absorption spectrum of the citrate prepolymer prepared according to Example 3.
FIG. 9 shows the mechanical properties of the suture-free anti-adhesion membrane prepared according to Examples 1 to 4.
FIG. 10 shows the self-adhesive performance of the suture-free anti-adhesion membrane prepared according to the steps of Examples 1 to 4.
FIG. 11 shows the cell compatibility of the suture-free anti-adhesion membrane prepared according to the steps of Examples 1 to 4.
FIG. 12 is an anti-protein adhesion effect image of the suture-free anti-adhesion membrane prepared according to Examples 1 to 4.
FIG. 13 is an in vivo self-adhesive effect image of the suture-free anti-adhesion membrane prepared according to Example 2 and Example 4.
FIG. 14 is an in vivo H&E-stained tissue section image of the suture-free anti-adhesion membrane prepared according to Example 4.
FIG. 15 is a real object image of the degradation of the suture-free anti-adhesion membrane prepared according to Example 4 in vivo.
FIG. 16 shows the synthesis equation for preparing citric acid-based prepolymer according to step 1 of Examples 5 to 7.
FIG. 17 is an effect image prepared according to the preparation method of Example 8.
FIG. 18 is an effect image prepared according to the preparation method of Example 9.
FIG. 19 shows the tensile strength of the suture-free anti-adhesion membrane prepared according to Example 10.
FIG. 20 shows the self-adhesive strength of the suture-free anti-adhesion membrane prepared according to Example 12 and Comparative Example 1. The prepared membrane material is soaked in PBS with pH=7.4 to observe its wet adhesion performance.
FIG. 21 shows the comparison of the self-adhesive performance of suture-free anti-adhesion membranes prepared according to Example 1 and Example 13. The thickness of the membrane material significantly affects the self-adhesive strength.

### DETAILED DESCRIPTION

The following examples are provided to better facilitate further understanding of the present application, and are not limited to the described optimal embodiments. These examples do not constitute restrictions on the content and scope of protection of the present application. Any product that is identical or similar to the present application, whether derived from the insights of the present application or by combining the present application with features of other existing technologies, falls within the scope of protection of the present application.

If specific experimental steps or conditions are not specified in the examples, the operations or conditions of the conventional experimental steps described in the literature within the field can be followed. If the manufacturer of the reagents or instruments used is not specified, they are all conventional reagent products that can be obtained commercially.

The embodiments of the present application disclose a method for preparing a self-adhesive material, which comprises: reacting component A and component B to form a citric acid-based prepolymer; the component A is at least one compound containing at least two functional groups which are capable of reaction and are at terminal groups or on side chains of the component A, wherein the functional groups are the same or different; the functional group is selected from at least one of hydroxyl, amino, sulfydryl, epoxy, carboxyl, acid anhydride, and organic substituents; the component B is citric acid and/or derivatives thereof; the reaction comprises a polycondensation reaction, a substitution reaction, or a transesterification reaction; and performing a cross-linking reaction of the citric acid-based prepolymer and isocyanate via a urethane bond or an amide bond. The research in the present application has found that isocyanate does not participate in the preparation process of polyester prepolymer. By directly crosslinking isocyanate with the citric acid-based prepolymer at the crosslinking points (urethane bonds or amide bonds) without demand for thermal crosslinking, the obtained material exhibits self-adhesive property. The main reason for this is that, the material primarily forms urethane bonds or amide bonds through crosslinking of isocyanates, which can enhance the hydrogen bonding interaction between the contact surfaces of the material to achieve self-adhesion. Therefore, when this material is applied to anti-adhesion membranes, the difficulty of surgical operation and the risk of infection can be reduced without suturing. Furthermore, this material also possesses satisfactory degradation rate, mechanical strength and histocompatibility.

In some embodiments, the isocyanate is selected from at least one of 1,6-hexamethylene diisocyanate, isophorone diisocyanate, toluene diisocyanate, lysine diisocyanate, naphthalene diisocyanate, 1,4-cyclohexane diisocyanate, 4,4'-diphenylmethane diisocyanate and 4,4'-methylenebis(cyclohexyl isocyanate). By directly crosslinking the citric acid-based prepolymer with isocyanate, the urethane bond or amide bond formed by crosslinking enhances the hydrogen bonding interaction between the contact surfaces of the material, resulting in good self-adhesion between the contact surfaces of the obtained material. This allows for suture-free fixation during application, greatly reducing the difficulty of surgical operation and the risk of infection. The aforementioned isocyanates can all achieve this function by adjusting the addition ratio with the prepolymer.

Furthermore, since the self-adhesive material need to possess a windable performance, being too hard or too brittle will affect their winding performance. Therefore, if the molecular structure of the selected isocyanate is too rigid or the amount used is excessive, it will lead to an increase in material strength, which is not conducive to winding. When implanted into the body, it is prone to cause a significant foreign body sensation and easily trigger inflammatory reactions. However, the molecular structure of the aforementioned isocyanate can ensure that the prepared self-adhesive material possesses a windable performance.

In a preferred embodiment, the isocyanate is selected from at least one of 1,6-hexamethylene diisocyanate, lysine diisocyanate and isophorone diisocyanate. Compared to diisocyanates with branched chains or cyclic structures, the molecular structures of 1,6-hexamethylene diisocyanate, lysine diisocyanate and isophorone diisocyanate exhibit less rigidity in their molecular chains. Therefore, when regulating the ratio, the upper limit of their addition amount is higher, allowing for the introduction of more hydrogen bonding structures, resulting in a corresponding enhancement of the self-adhesive performance of the material.

In some embodiments, when the citric acid-based prepolymer is in a solid state, before conducting the cross-linking reaction of the urethane bond or the amide bond, the citric acid-based prepolymer is dissolved in an organic solvent to obtain a citric acid-based prepolymer solution; the isocyanate is added to the citric acid-based prepolymer solution to obtain a mixed solution, and then the cross-linking reaction of the urethane bond or the amide bond is performed; the mass percentage of the citric acid-based prepolymer in the citric acid-based prepolymer solution is in a range from 30% to 60%; the amount of the isocyanate added is greater than 0% and less than or equal to 50% by mass percentage of the citric acid-based prepolymer. Furthermore, the mass percentage of the citric acid-based prepolymer in the citric acid-based prepolymer solution may be any one of 30%, 33%, 35%, 38%, 40%, 43%, 45%, 48%, 50%, 53%, 55%, 58% and 60%, or a range between any two of the values; the amount of isocyanate added is any one of 0.01%, 0.1%, 0.5%, 1%, 5%, 8%, 10%, 13%, 15%, 18%, 20%, 23%, 25%, 28%, 30%, 33%, 35%, 38%, 40%, 45%, 48% and 50% by mass percentage of the citric acid-based prepolymer, or a range between any two of the values. By controlling the ratio of the citric acid-based prepolymer to isocyanate, the adhesion of the material can be improved. The reason is that the urethane bond or amide bond formed by the direct crosslinking of isocyanate with the citric acid-based prepolymer enhances the hydrogen bonding interaction between the contact surfaces of the material, resulting in good self-adhesion between the contact surfaces of the obtained material. Therefore, the ratio of the citric acid-based prepolymer to isocyanate will directly affects the content of hydrogen bonds, thereby affecting the adhesive performance of the material. Furthermore, since the self-adhesive material need to possess a windable performance, being too hard or too brittle will affect their winding performance. Therefore, if the amount of the selected isocyanate used is excessive, it will lead to an increase in material strength, which is not conducive to winding. When implanted into the body, it is prone to cause a significant foreign body sensation and easily trigger inflammatory reactions. By selecting the above-mentioned ratio, a self-adhesive material with superior windable performance can be prepared.

In some embodiments, the prepolymer prepared with citric acid as component B is in a solid state and requires an anhydrous organic solvent for dissolution. Examples of suitable organic solvents include acetone, dioxane, cyclohexane or isopropyl ether, etc. The mass ratio of prepolymer to organic solvent is in a range of 30% to 60%, and the optional organic solvent is dioxane. In one embodiment, the prepolymer prepared with citrate as component B is in a liquid state and does not require an organic solvent for dissolution.

In some embodiments, the cross-linking reaction of the urethane bond or the amide bond is carried out at room temperature for a duration ranging from 0 h to 24 h, with the duration being greater than 0 h, ensuring that the added organic solvent is completely evaporated. Furthermore, the reaction time may be any one of > 0 min, 10 min, 20 min, 30 min, 40 min, 50 min, 60 min, 1.5 h, 2 h, 2.5 h, 3 h, 3.5 h, 4 h, 4.5 h, 5 h, 5.5 h, 6 h, 6.5 h, 7 h, 7.5 h, 8 h, 8.5 h, 9 h, 10 h, 11 h, 12 h, 13 h, 14 h, 15 h, 16 h, 17 h, 18 h, 19 h, 20 h, 21 h, 22 h, 23 h and 24 h, or a range between any two of the values. Using the method of the present application, the temperature is room temperature without the demand for heating, and the reaction is rapid. The temperature range of the room temperature is in a range from 10°C to 30°C, including but not limited to 10°C, 15°C, 20°C, 25°C and 30°C.

In some embodiments, a step of preparing the self-adhesive material into a membrane, comprising spin coating, solution casting, or solution casting to form a membrane from a mixed solution of the citric acid-based prepolymer and the isocyanate during the cross-linking reaction of the urethane bond or the amide bond.

In some embodiments, the spin coating is performed at a rotation speed ranging from 1000 rpm to 3000 rpm and for a duration ranging from 0 min to 3 min, with the duration being greater than 0 min. Furthermore, the rotation speed may be any one of 1000 rpm, 1100 rpm, 1200 rpm, 1500 rpm, 1800 rpm, 2000 rpm, 2200 rpm, 2500 rpm, 2800 rpm and 3000 rpm, or a range between any two of the values; the duration may be any one of 0.01 min, 0.1 min, 0.5 min, 0.8 min, 1 min, 1.2 min, 1.5 min, 1.8 min, 2.0 min, 2.2 min, 2.5 min, 2.8 min and 3 min, or a range between any two of the values.

Furthermore, in some embodiments, after spin coating, solution casting, or solution casting to form a membrane, further crosslinking is performed under ultraviolet irradiation, thereby enabling the preparation of photocrosslinkable or 3D printable membrane materials. The ultraviolet irradiation conditions may be 365 nm ultraviolet irradiation for 1 min to 3 min.

In some embodiments, the component A is a hydroxy compound, including but not limited to polyols and ethers. The polyols include, for example, diols. The diols include but are not limited to C2-C20, C2-C12 or C2-C6 aliphatic alkanediols, including α,ω-n-alkane diols or α,ω-olefin diols. Furthermore, the hydroxy compounds include but are not limited to ethylene glycol, hexanediol, octanediol, polyethylene glycol, bis(4-hydroxyphenyl) disulfide, resveratrol, bis(2-hydroxyethyl) disulfide, isosorbide, xylitol, hydroxyacetate or N-methyldiethanolamine.

In some embodiments, when the component A is an amino acid or other compounds containing amino group or sulfydryl group, including but not limited to serine, phosphoserine, cysteine, glutamine, polyaniline, etc.

In some embodiments, when the component A is an epoxy compound, including but not limited to epoxidized soybean oil, epoxy resin, epichlorohydrin, lactide, etc.

In some embodiments, when the component A is a compound containing the carboxyl group or acid anhydride, including but not limited to folic acid, tartaric acid, adipic acid, succinic acid, fumaric acid, salicylic acid, acetic anhydride, maleic anhydride, etc.

In some embodiments, when the component A is a compound containing the organic substituents, including but not limited to polyphosphazene, etc.

In some embodiments, the citric acid and/or derivatives thereof include but are not limited to citric acid, trimethyl citrate, triethyl citrate, tributyl citrate, acetyl triethyl citrate, stearyl monoglyceride citrate and citric acid luminescent molecules (such as CA-Cys).

In some embodiments, the component A is either a hydrophobic compound or a hydrophilic compound. When component A is a hydrophilic compound (such as polyethylene glycol), the prepared material is a hydrophilic material. When component A is a hydrophobic compound (such as octanediol), the prepared material is a hydrophobic material.

In some embodiments, when the component A containing amino acids reacts with citric acid, a fluorophore is capable of being generated, enabling the preparation of materials with fluorescence functionality for applications such as biological imaging and localization.

In some embodiments, the mechanical strength of the material may be regulated by adjusting the ratio of citric acid-based prepolymer or isocyanate, or the molecular structure of isocyanate, which can significantly improve the strength of the tissue anti-adhesion membrane.

In some embodiments, the functional groups on the side chains of the citric acid-based prepolymer can also be loaded with drugs such as antibacterial, promoting nerve regeneration or promoting tissue repair, or encapsulate various drugs during the crosslinking process to adapt to repairs of different defect sites, thereby enabling the preparation of materials with drug delivery functions, such as tissue anti-adhesion membranes.

In some embodiments, component A, such as a dicarboxylic acid, an unsaturated fatty acid or acid anhydride, can be reacted with citric acid to prepare a photocrosslinkable citric acid-based prepolymer. The prepolymer may be used to fabricate materials suitable for different sizes and shapes of defect sites through 3D printing, and may be utilized to prepare tissue anti-adhesion membranes with photocrosslinking or 3D printing functions.

In some embodiments, citric acid is used as component B, with a molar ratio of 1 to 2 relative to a compound which contains hydroxyl group, amino group, sulfydryl group, epoxy group, carboxyl group, acid anhydride, organic substituent and the like in the side chains or terminal groups of component A. The polymerization temperature is in a range from 140°C to 160°C, the polymerization time is in a range from 0 h to 6 h and greater than 0 h, and the rotation speed is in a range from 100 rpm to 1000 rpm. Furthermore, the polymerization temperature may be any one of 140°C, 142°C, 145°C, 148°C, 150°C, 153°C, 155°C, 158°C and 160°C, or a range between any two of the values. The rotation speed may be any one of 100 rpm, 200 rpm, 500 rpm, 600 rpm, 800 rpm and 1000 rpm, or a range between any two of the values. The reaction time may be any one of 0.1 min, 1 min, 5 min, 10 min, 20 min, 0.5 h, 1 h, 2 h, 3 h, 4 h, 5 h and 6 h, or a range between any two of the values.

In some embodiments, citrate is used as component B, with a molar ratio of 1 to 2 to the compound with hydroxyl groups on the side chains or terminal groups of component A. The polymerization temperature is in a range from 140°C to 160°C, the polymerization time is in a range from 0 h to 48 h and greater than 0 h, and the rotation speed is in a range from 100 rpm to 1000 rpm. Furthermore, the polymerization temperature may be any one of 140°C, 142°C, 145°C, 148°C, 150°C, 153°C, 155°C, 158°C and 160°C, or a range between any two of the values. The rotation speed may be any one of 100 rpm, 200 rpm, 500 rpm, 600 rpm, 800 rpm and 1000 rpm, or a range between any two of the values. The reaction time may be any one of 0.1 min, 1 min, 5 min, 10 min, 20 min, 0.5 h, 1 h, 2 h, 4 h, 6 h, 8 h, 10 h, 12 h, 14 h, 16 h, 18 h, 20 h, 22 h, 24 h, 26 h, 28 h, 30 h, 32 h, 34 h, 36 h, 38 h and 48 h, or a range between any two of the values.

The embodiments of the present application provide the use of the self-adhesive material in any of the following:
(1) the use of preparing a suture-free and/or an anti-adhesion biomembrane;
(2) the use of the suture-free and/or the anti-adhesion biomembrane as a tissue anti-adhesion membrane for tendons, abdominal cavity, pelvic cavity, nerves, and spinal site.

The alcohols used in the following examples, such as hexanediol, octanediol, and cyclohexane, and cysteine are of analytical grade.

### Example 1

Provided is an example of preparing citric acid-based polymer via polycondensation of citric acid with a polyol, followed by crosslinking with a diisocyanate to form a membrane.
(1) 10 g of citric acid and 7 g of 1,6-hexanediol were added into a flask and reacted at 140°C for 3 h, with the rotation speed of 800 rpm. The reactant was precipitated with deionized water, and the citrate prepolymer was obtained through lyophilization;
(2) the mass of the prepolymer was quantified accurately, and cyclohexane was added to obtain a prepolymer solution with a mass fraction of 50%;
(3) 1,6-hexamethylene diisocyanate, 20% by mass percentage of the prepolymer, was added to 1 g of prepolymer solution (i.e., 100 mg of 1,6-hexamethylene diisocyanate was added to 0.5 g of prepolymer), and spin coated on a spin coater at a speed of 1,000 rpm for 1 min to form a membrane. After the membrane was dried, it was removed to obtain a suture-free anti-adhesion membrane.

The obtained suture-free anti-adhesion membrane was subjected to various index tests, and the test results were as follows:

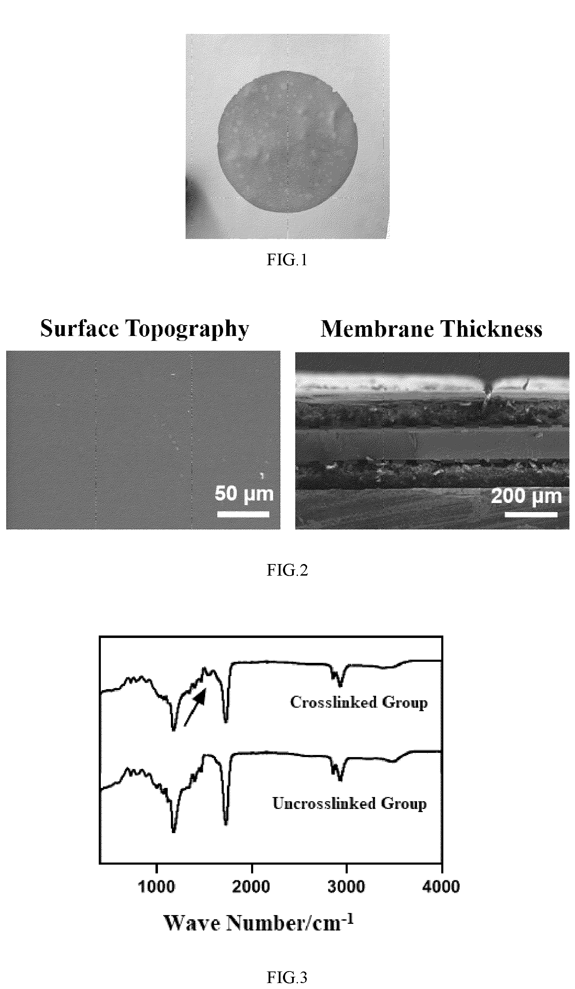

FIG. 1 is a real-life rendering of the suture-free anti-adhesion membrane prepared according to step (3) of Example 1.
FIG. 2 is a scanning electron microscopy image of the surface and thickness of the suture-free anti-adhesion membrane prepared according to step (3) of Example 1. As can be seen in the figure, the membrane prepared by this preparation method has a dense and smooth surface without pore structure, enabling it effectively to prevent the growth of fibroblasts.
FIG. 3 is an infrared spectrum of the suture-free anti-adhesion membrane prepared according to step (3) of Example 1 (crosslinked group) and the citrate prepolymer prepared according to step (1) of Example 1 (uncrosslinked group). The image reveals a new characteristic peak at 1535 cm⁻¹ in the crosslinked group, which belongs to the characteristic absorption peak of -NH- in carbamate, indicating that the membrane is prepared through chemical crosslinking.

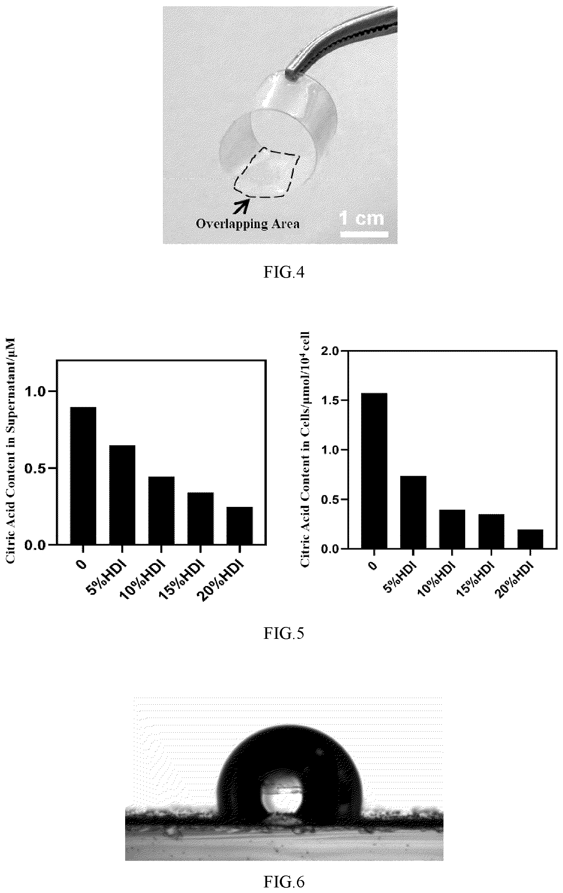

FIG. 4 is a self-adhesive effect diagram of the suture-free anti-adhesion membrane prepared according to Example 1. The image shows that the membranes can be overlapped together by hydrogen bonding with each other, causing self-winding and eliminating the experimental step of suturing.
FIG. 5 is a statistical graph showing the citric acid content released from the suture-free anti-adhesion membrane prepared from the prepolymer according to Example 1 using different mass ratios of isocyanate, as well as the intracellular citric acid content after co-incubation. The left side one of FIG. 5 shows the citric acid content released from the suture-free anti-adhesion membrane into the supernatant. The right side one of FIG. 5 shows the citric acid content in the cells after co-incubation of the supernatant with cells. The specific method is as follows: according to step (3) of Example 1, different mass percentages (0%, 5%, 10%, 15%, 20%) of 1,6-hexamethylene diisocyanate is added to the prepolymer solution prepared in step (2) for crosslinking to form a self-adhesive anti-adhesion membrane; a membrane with a diameter of 8 mm is soaked in 1 mL of PBS (pH=7.4) and placed in an incubator at 37°C for 24 h to obtain the leaching solution. Then, the citric acid content in the supernatant of the leaching solution is detected. Under the same conditions, leached with culture medium, and co-culture the supernatant with mice embryonic fibroblasts (NIH/3T3). That is, 10,000 cells are added to each well of 96T and co-cultured for 24 h. After 24 h, 100 µL of leaching solution is replaced per well and continue co-cultured for another 24 h, and then washed with PBS (pH=7.4), after lysis and centrifugation, the intracellular citric acid content is measured. The results show that membranes with a high degree of cross-linking degrade slowly and released correspondingly less citric acid content, leading to a corresponding decrease in intracellular citric acid content.

### Example 2

The difference between this example and Example 1 was that 1,6-hexamethylene diisocyanate was replaced with lysine diisocyanate in equal mass to prepare a strength-adjustable membrane.

The obtained suture-free anti-adhesion membrane was subjected to various index tests, and the test results were as follows:
The obtained suture-free anti-adhesion membrane was subjected to water contact angle testing according to the national standard GB/T 30693-2014. FIG. 6 was the water contact angle image of the suture-free anti-adhesion membrane prepared according to Example 2. The results indicated that the water contact angle of the membrane is 109.5°, indicating that the introduction of long alkyl chains of hexanediol rendered the membrane hydrophobic and had an anti-fouling effect.

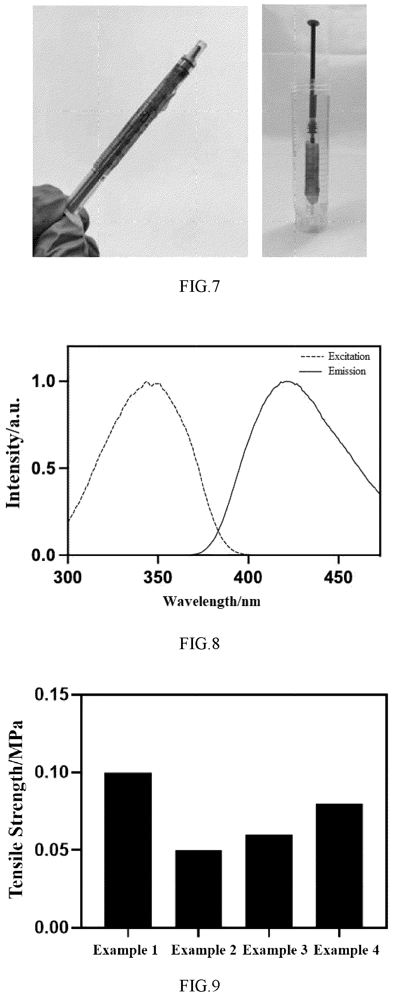

FIG. 7 illustrated the self-adhesive effect image and adhesion stability test of the suture-free anti-adhesion membrane prepared according to Example 2. The testing method was as follows: the suture-free anti-adhesion membrane was wrapped around a 1 mL syringe to simulate the wrapping effect on a tendon site. The length of the material membrane was slightly longer than the circumference of the syringe. Through the self-lapping of the membrane, the membrane material can be stably wrapped around the outer surface of the syringe without detachment (see the left side of FIG. 7). Further, the entire membrane was soaked in PBS (pH=7.4) and placed in a shaker at 37°C for one month, and then the results were observed. The results showed that (see the right side of FIG. 7) even in a wet environment, the membrane still maintained a good wrapping effect on the syringe surface without detachment, indicating that the membrane had good long-term stability in a wet environment and potential for application in a wet environment in vivo.

### Example 3

An example of preparing a citric acid-based polymer through a polycondensation of citric acid and amino acid, and crosslinking it with diisocyanate to prepare an imageable membrane.
(1) 10 g of citric acid and 6 g of cysteine were added into a flask and reacted at 140°C for 4 h, with the rotation speed set to 800 rpm. The reactant was precipitated with deionized water, and the citrate prepolymer was obtained through lyophilization;
(2) the mass of the prepolymer was quantified accurately, and cyclohexane was added to obtain a prepolymer solution with a mass fraction of 50%;
(3) 1,6-hexamethylene diisocyanate, 20% by mass percentage of the prepolymer, was added to 1 g of prepolymer solution. And spin coated on a spin coater at a speed of 1,000 rpm for 1 min to form a membrane. After the membrane was dried, it was removed to obtain a suture-free anti-adhesion membrane.

The citrate prepolymer obtained in step (1) was subjected to fluorescence absorption spectroscopy detection with an excitation wavelength of 350 nm and an emission wavelength of 420 nm. The detection results were shown in FIG. 8, which represented the fluorescence absorption spectrum of the citrate prepolymer prepared according to the steps of Example 3. This demonstrated that the reaction of molecules containing amino groups, such as amino acids and citric acid, can generate a fluorophore. By using different amino acids or molecules containing amino groups, a series of photoluminescent fluorescent molecules can be obtained, which can be used to prepare a series of citric acid-based degradable macromolecule photoluminescent suture-free anti-adhesion membranes with tunable excitation wavelengths and emission wavelengths (visible light-near infrared) for applications such as biological images.

### Example 4

Provided is an example of preparing citric acid-based polymer via transesterification of a citrate with a polyol, followed by crosslinking with a diisocyanate to form a membrane.
(1) 10 g of triethyl citrate and 6 g of 1,6-hexanediol were added into a flask and reacted at 140°C for 24 h, with the rotation speed set to 800 rpm. The reactant was precipitated with deionized water, and a liquid citrate prepolymer was obtained through lyophilization;
(2) 1,6-hexamethylene diisocyanate, 20% by mass percentage of the prepolymer, was added to 1 g of prepolymer solution, and spin coated on a spin coater at a speed of 1,000 rpm for 1 min to form a membrane. After the membrane was dried, it was removed to obtain a suture-free anti-adhesion membrane.

### Example 5

Provided is an example of preparing citric acid-based polymer via polycondensation of citric acid, a polyol and an acid anhydride, followed by crosslinking with a diisocyanate under ultraviolet light to form a membrane.
(1) 8 g of citric acid, 2 g of maleic anhydride, and 7 g of octanediol were added into a flask and reacted at 140°C for 3 h, with the rotation speed set to 800 rpm. The reactant was precipitated with deionized water, and the citrate prepolymer was obtained through lyophilization;
(2) the mass of the prepolymer was quantified accurately, and cyclohexane was added to obtain a prepolymer solution with a mass fraction of 50%;
(3) 1,6-hexamethylene diisocyanate, 5% by mass percentage of the prepolymer, was added to 1 g of prepolymer solution, and spin coated on a spin coater at a speed of 1,000 rpm for 1 min to form a membrane, then irradiated with 365 nm ultraviolet light for 3 min for further crosslinking. After the membrane was dried, it was removed to obtain a suture-free anti-adhesion membrane.

### Example 6

Provided is an example of preparing citric acid-based polymer via polycondensation of citric acid with a polyamine, followed by crosslinking with a diisocyanate to form a membrane.
(1) 10 g of citric acid and 6 g of hexanediamine were added into a flask and reacted at 140°C for 3 h, with the rotation speed set to 800 rpm. The reactant was precipitated with deionized water, and the citrate prepolymer was obtained through lyophilization;
(2) the mass of the prepolymer was quantified accurately, and cyclohexane was added to obtain a prepolymer solution with a mass fraction of 50%;
(3) 1,6-hexamethylene diisocyanate, 10% by mass percentage of the prepolymer, was added to 1 g of prepolymer solution, and spin coated on a spin coater at a speed of 1,000 rpm for 1 min to form a membrane. After the membrane was dried, it was removed to obtain a suture-free anti-adhesion membrane.

### Example 7

Provided is an example of preparing citric acid-based polymer via polycondensation of citric acid with a polycarboxylic acid, followed by crosslinking with a diisocyanate to form a membrane.
(1) 10 g of citric acid and 7 g of adipic acid were added into a flask and reacted at 140°C for 3 h, with the rotation speed set to 800 rpm. The reactant was precipitated with deionized water, and the citrate prepolymer was obtained through lyophilization;
(2) the mass of the prepolymer was quantified accurately, and cyclohexane was added to obtain a prepolymer solution with a mass fraction of 50%;
(3) 1,6-hexamethylene diisocyanate, 10% by mass percentage of the prepolymer, was added to 1 g of prepolymer solution, and spin coated on a spin coater at a speed of 1,000 rpm for 1 min to form a membrane. After the membrane was dried, it was removed to obtain a suture-free anti-adhesion membrane.

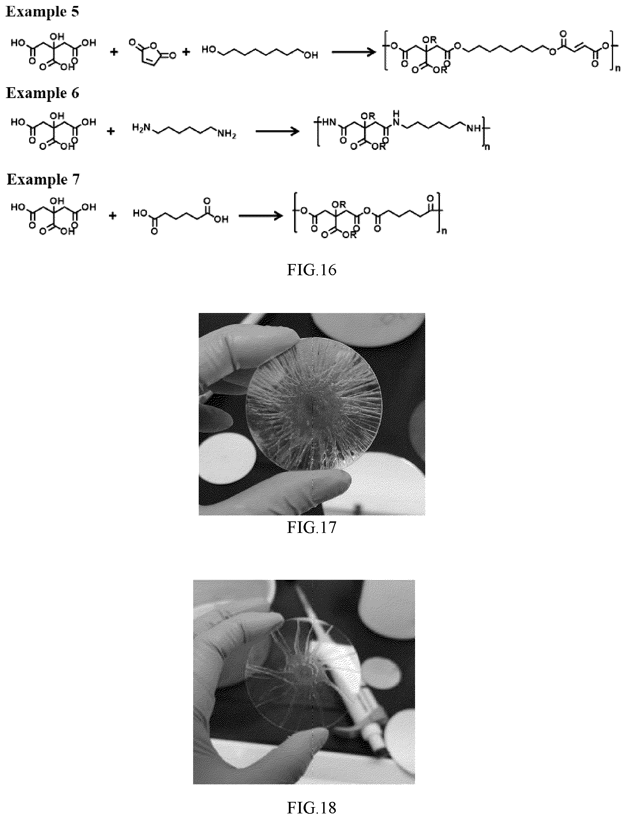

FIG. 16 showed the synthesis equation for preparing citric acid-based prepolymer according to step (1) of Examples 5 to 7.

### Example 8

A suture-free anti-adhesion membrane was prepared according to the method of Example 2, with the difference being that the membrane formation through spin coating at a speed of 500 rpm.

Result: the membrane state after spin coating was observed, due to the hydrophobicity and relatively high viscosity of the prepolymer solution, it was difficult to evenly coat the coating at a low rotation speed, as shown in FIG. 17.

### Example 9

A suture-free anti-adhesion membrane was prepared according to the method of Example 4, with the difference being that the proportion of 1,6-hexamethylene diisocyanate added accounts for 100% of the prepolymer.

Result: due to the excessively high proportion of isocyanate, the reaction rate between the prepolymer and isocyanate was too fast, resulting in rapid curing of the spin-coating solution, making it difficult to spin coat, as shown in FIG. 18.

### Example 10

The difference between this example and Example 1 was that toluene diisocyanate was used to replace 1,6-hexamethylene diisocyanate in equal mass.

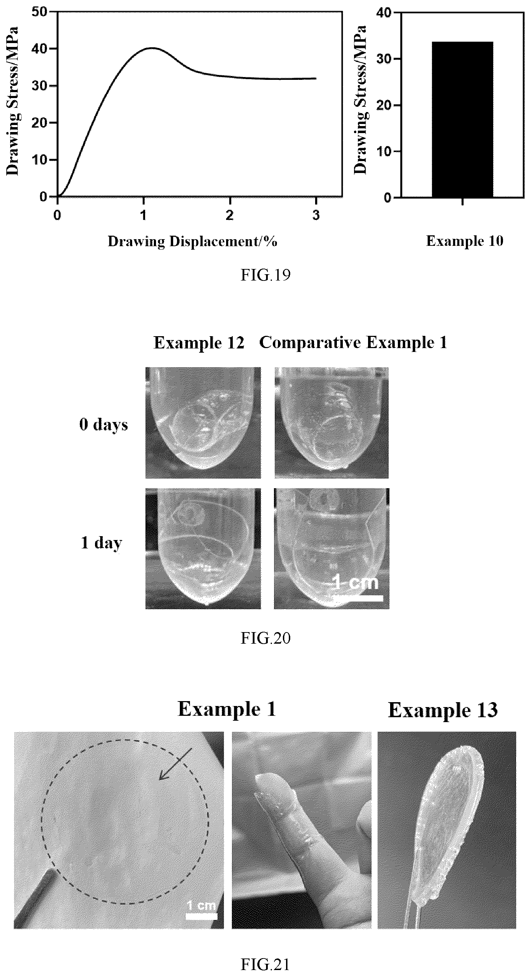

Result: due to the excessively rigid molecular structure of toluene diisocyanate, the resulting membrane exhibited a strength of up to 33.68 Mpa, making it impossible to bend for self-wrapping. Furthermore, when implanted into the body, it did not match the surrounding tissues, which could easily trigger inflammatory reactions, making it unsuitable for in vivo applications, as shown in FIG. 19.

### Example 11

A suture-free anti-adhesion membrane was prepared according to the method of Example 1, with the difference being that polyethylene glycol 1000 was used to replace 1,6-hexanediol in equal mass, but the membrane was unable to be formed under this crosslinking ratio.

### Example 12

A suture-free anti-adhesion membrane was prepared according to the method of Example 1, with the difference being that: (3) 1,6-hexamethylene diisocyanate, 5% by mass percentage of the prepolymer, was added to 1 g of prepolymer solution.

Result: due to the excessively low crosslinking ratio, there were not enough hydrogen bonding sites for the occurrence of self-adhesion performance, resulting in relatively poor self-adhesion performance of the material. Self-adhesion effect was generated in the dry state, but once soaked in PBS with pH=7.4, the self-adhesion sites underwent displacement and detachment, thereby losing the self-adhesion effect, as shown in FIG. 20.

### Example 13

A suture-free anti-adhesion membrane was prepared according to the method of Example 1, with the difference being that the system of the membrane-forming solution was expanded by 5 times, i.e., 1,6-hexamethylene diisocyanate, 20% by mass percentage of the prepolymer, was added to 5 g of prepolymer solution (i.e., 500 mg of hexamethylene diisocyanate was added to 2.5 g of prepolymer), and then spin coated to form a membrane.

Results: The coating thickness was measured using a vernier caliper and was about 720 µm. The excessive thickness of the membrane material resulted in excessively high surface tension, and the hydrogen bonding interaction at the membrane adhesion joint could not maintain good self-adhesion effects, leading to the loss of self-adhesion performance of the membrane material. Meanwhile, due to the relatively thick membrane material, there was a noticeable foreign body sensation when in contact with skin tissue, making it unsuitable for internal use within tissues. However, the membrane material in Example 1 had a coating thickness of about 100 µm, which allowed effective self-adhesion at the finger site, and it did not crack when bent, demonstrating good self-adhesion performance. Therefore, the thickness of the membrane material was closely related to its self-adhesion performance, as shown in FIG. 21.

### Comparative Example 1

A suture-free anti-adhesion membrane was prepared according to the method of Example 1, with the difference being that isocyanate crosslinking was not employed, and thermal crosslinking was employed alone. That is, 1 g of prepolymer solution was taken and spin coated on a spin coater at a speed of 1,000 rpm for 1 min to form a membrane. The membrane was placed in an 80°C oven for thermal crosslinking. After the membrane was dried, it was removed to obtain a suture-free anti-adhesion membrane.

Result: when employing thermal crosslinking, the membrane material was only cross-linked by ester bonds and lacks hydrogen bonding sites, resulting in poor self-adhesion performance of the material, as shown in FIG. 20.

### Experimental Example 1

### This Experimental Example examined the various properties of the suture-free anti-adhesion membranes prepared in Examples 1 to 4

### (1) Mechanical properties

The testing method was as follows: the membrane material was made into a spline with a width of 1.5 cm and a length of 4 cm, and placed on a universal testing machine. It was clamped at a position 0.5 cm above and below, and stretched at a rate of 50 mm/min. The results were shown in FIG. 9, which illustrated the mechanical properties of the suture-free anti-adhesion membrane prepared according to Examples 1 to 4. Its tensile strength ranged from 50 Kpa to 100 Kpa, which can be adjusted by regulating the molecular structure of isocyanate. It matched the mechanical properties of commercially available materials and was able to fulfill the supporting function at the site of tendon injury.

### (2) Self-adhesion

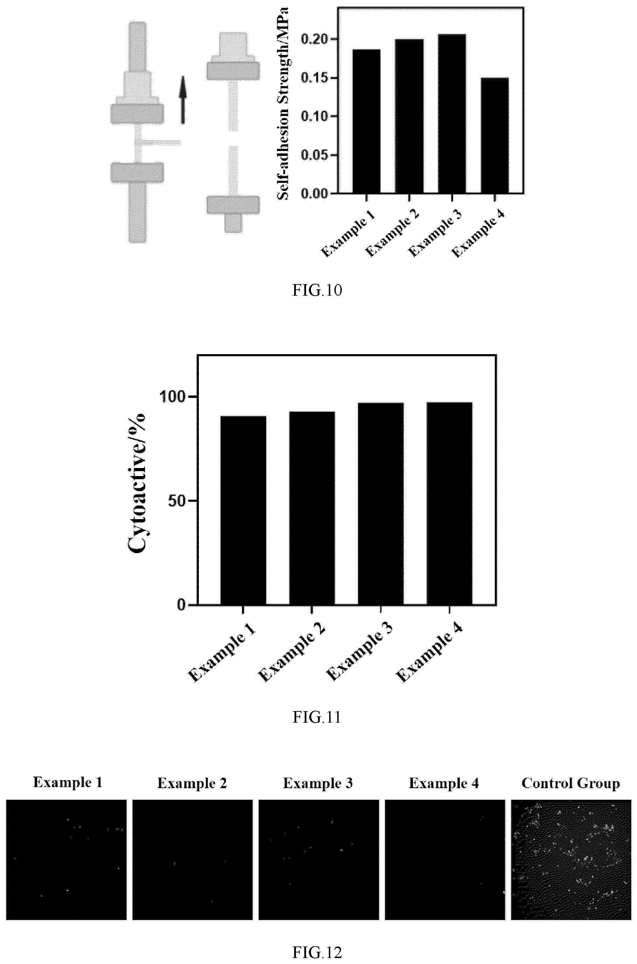

The testing method was as follows: two pieces of the same suture-free anti-adhesion membrane prepared were used, and the two membranes were overlapped face to face, in a T-shape configuration, uniform pressure was applied on the entire overlapping face, with a recommended applying pressure of up to 1 Mpa, as shown on the left side of FIG. 10, and the strength of the adhesion joint was measured, referring to the national standard GB/T 2791-1995.

The right side of FIG. 10 shows the self-adhesive performance of the suture-free anti-adhesion membrane prepared according to the steps of Examples 1 to 4. The results indicated that the prepared membrane had self-adhesive properties with a strength ranging from 0.14 Mpa to 0.22 Mpa, and its adhesive strength can be adjusted by regulating the molecular structure of isocyanate.

### (3) Cytotoxicity

The detection method was as follows: the suture-free anti-adhesion membrane (8 mm diameter) prepared in each example was taken and soaked in 1 mL of DMEM culture medium, and placed in a 37°C incubator for 24 h to obtain the leaching solution. Then, an equal volume of cell supernatant was replaced with leaching solution and co-cultured with mice embryonic fibroblasts (NIH/3T3). That is, 10,000 cells were added to each well of 96T and co-cultured for 24 h. After 24 h, 100 µL of leaching solution was replaced per well and continue co-cultured for another 24 h. Then, the absorbance value at OD₆₀₀ nm was measured using CCK8, wherein the fully live group was replaced with cell culture medium, and the fully dead group was replaced with sterile water. The formula for calculating cell survival rate was as follows: 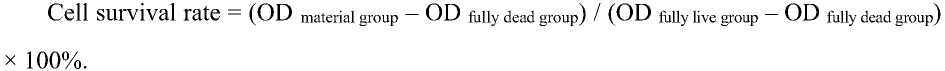 Cell survival rate = (OD material group - OD fully dead group) / (OD fully live group - OD fully dead group) × 100%.

FIG. 11 showed the cytotoxicity of the suture-free anti-adhesion membrane prepared according to the steps of Examples 1 to 4. The results revealed that the cell survival rate in all groups was higher than 70%, this indicated that the citric acid-based suture-free anti-adhesion membrane prepared using this preparation method exhibited good biocompatibility and can be used for further characterization in vivo.

### (4) Anti-protein adhesion effect

The detection method was as follows: the membranes with a diameter of 8 mm prepared in each example were placed into the wells of a 48-well plate. A solution of FITC-labeled BSA was prepared at 2 mg/mL using PBS, and 200 microliters were added to each well. A control group was set up simultaneously using commercially available membrane material (Sepramembrane^{®}). After co-incubation at 37°C for 2 h, the membranes were washed with PBS and observed under a fluorescence microscope.

FIG. 12 is the anti-protein adhesion effect image of the suture-free anti-adhesion membrane prepared according to the steps of Examples 1 to 4. The results demonstrated that the citric acid-based suture-free anti-adhesion membrane prepared by this preparation method can effectively prevent protein adhesion on its surface, exhibiting good anti-adhesion effect.

### Experimental Example 2

### This experimental example examined the anti-adhesion effect of suture-free anti-adhesion membrane in a tendon injury model

Application in tendon injury model: SD rats weighing about 200 g to 250 g were used for in vivo tendon injury animal modeling. Rats were anesthetized with pentobarbital sodium. After removing the hair on the surface of the Achilles tendon, they were disinfected with iodophor. The skin was incised longitudinally along the Achilles tendon, and the Achilles tendon site was transected and disconnected. A membrane with a size of 1.0 cm (width) × 2.0 cm (length) was used for wrapping. Then, the skin was sutured and disinfected again. Said membrane was the suture-free anti-adhesion membrane prepared according to the steps of Examples 2 and 4. The control group was sutured with surgical thread.

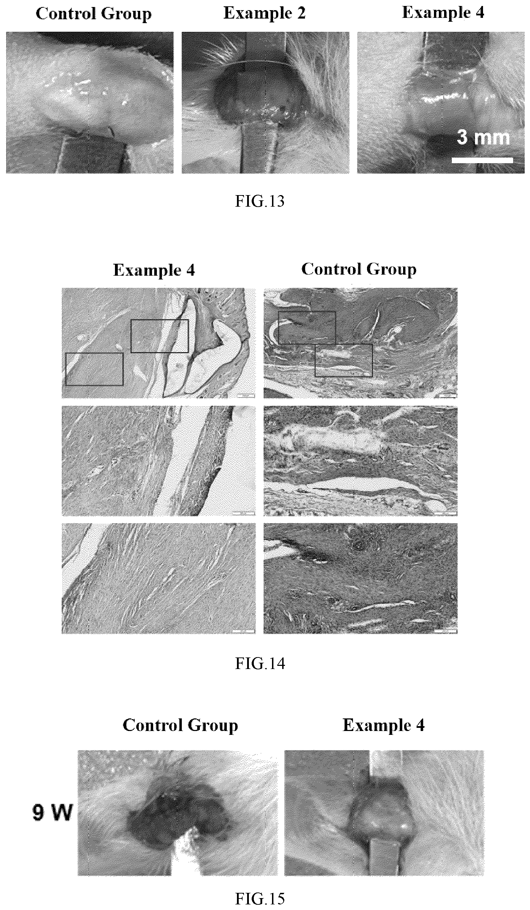

FIG. 13 is the wrapping effect image of the suture-free anti-adhesion membrane prepared according to Examples 2 and 4 on the tendon injury site of rats. The results showed that the membranes prepared by the methods in Examples 2 and 4 can effectively wrap the tendon injury site without suturing, thereby significantly reducing surgical time and avoiding the risk of infection. However, the control group required surgical suturing to ensure anastomosis and repair.

FIG. 14 is an H&E-stained tissue section of the suture-free anti-adhesion membrane prepared according to Example 4, after being wrapped around the tendon injury site of rats for one day. The results indicated that a large number of inflammatory cell infiltrations occurred in the control group, while the membrane prepared by the method in Example 4 exhibited good biocompatibility in vivo. Additionally, there were a clear gap between the tendon and muscle, indicating that the citric acid-based suture-free anti-adhesion membrane has good anti-adhesion effects in vivo and can effectively prevent the occurrence of fibrous adhesion phenomenon.

FIG. 15 is a general observation image of the suture-free anti-adhesion membrane prepared according to Example 4 after being wrapped around the tendon injury site of rats for 9 weeks. The results indicated that the membrane was completely degraded at nine weeks, demonstrating that the citric acid-based anti-adhesion membrane prepared by this method was fully degradable in vivo, exhibited good biocompatibility, and did not accumulate in human internal tissues or organs.

Obviously, the above examples are merely examples provided for clear illustration, and are not intended to limit the embodiments. For those ordinary skilled in the art, other variations or modifications in different forms can be made based on the above description. It is neither necessary nor feasible to exhaust all embodiments here. The obvious variations or modifications derived from this are still within the scope of protection of the present invention.

## Claims

1. A method for preparing a self-adhesive material, wherein comprising:
reacting component A and component B to form a citric acid-based prepolymer; wherein the component A is at least one compound containing at least two functional groups which are capable of reaction and are at terminal groups or on side chains of the component A, the functional groups are the same or different; the functional group is selected from at least one of hydroxyl, amino, sulfydryl, epoxy, carboxyl, acid anhydride, and organic substituents; the component B is citric acid and/or derivatives thereof; the reaction comprises a polycondensation reaction, a substitution reaction, or transesterification reaction; and
performing a cross-linking reaction of the citric acid-based prepolymer and isocyanate via a urethane bond or an amide bond.

2. The method for preparing a self-adhesive material according to claim 1, wherein the isocyanate is selected from at least one of 1,6-hexamethylene diisocyanate, isophorone diisocyanate, toluene diisocyanate, lysine diisocyanate, naphthalene diisocyanate, 1,4-cyclohexane diisocyanate, 4,4'-diphenylmethane diisocyanate and 4,4'-methylenebis(cyclohexyl isocyanate).

3. The method for preparing a self-adhesive material according to claim 2, wherein the isocyanate is selected from at least one of 1,6-hexamethylene diisocyanate, lysine diisocyanate and isophorone diisocyanate.

4. The method for preparing a self-adhesive material according to any one of claims 1 to 3, wherein when the citric acid-based prepolymer is in a solid state, before conducting the cross-linking reaction of the urethane bond or the amide bond, the citric acid-based prepolymer is dissolved in an organic solvent to obtain a citric acid-based prepolymer solution; the isocyanate is added to the citric acid-based prepolymer solution to obtain a mixed solution, and then the cross-linking reaction of the urethane bond or the amide bond is performed;
a mass percentage of the citric acid-based prepolymer in the citric acid-based prepolymer solution is in a range from 30% to 60%; and
the amount of the isocyanate added is greater than 0% and less than or equal to 50% by mass percentage of the citric acid-based prepolymer.

5. The method for preparing a self-adhesive material according to any one of claims 1 to 3, wherein the cross-linking reaction of the urethane bond or the amide bond is carried out at room temperature for a duration ranging from 0 hours to 24 hours, with the duration being greater than 0 hours.

6. The method for preparing a self-adhesive material according to any one of claims 1 to 3, wherein a step of preparing the self-adhesive material into a membrane, comprising spin coating, solution casting, or solution casting to form a membrane from a mixed solution of the citric acid-based prepolymer and the isocyanate during the cross-linking reaction of the urethane bond or the amide bond.

7. The method for preparing a self-adhesive material according to claim 6, wherein the spin coating is performed at a rotation speed ranging from 1000 rpm to 3000 rpm and for a duration ranging from 0 min to 3 min, with the duration being greater than 0 min;
and/or, the thickness of the membrane is in a range from 10 µm to 500 µm.

8. The method for preparing a self-adhesive material according to any one of claims 1 to 3, wherein the component A is a hydrophobic compound or a hydrophilic compound;
and/or, when the component A is a hydroxyl compound, including but not limited to ethylene glycol, hexanediol, octanediol, polyethylene glycol, bis(4-hydroxyphenyl) disulfide, resveratrol, bis(2-hydroxyethyl) disulfide, isosorbide, xylitol, hydroxyacetate or N-methyldiethanolamine;
and/or, when the component A is an amino acid or other compounds containing the amino group or the sulfydryl group, including but not limited to serine, phosphoserine, cysteine, glutamine or polyaniline;
and/or, when the component A is an epoxy compound, including but not limited to epoxidized soybean oil, epoxy resin, epichlorohydrin or lactide;
and/or, when the component A is a compound containing the carboxyl group or the acid anhydride, including but not limited to folic acid, tartaric acid, adipic acid, succinic acid, fumaric acid, salicylic acid, acetic anhydride or maleic anhydride;
and/or, when the component A is a compound containing the organic substituents, including but not limited to polyphosphazene;
and/or, the citric acid and/or derivatives thereof include but are not limited to citric acid, trimethyl citrate, triethyl citrate, tributyl citrate, acetyl triethyl citrate, stearyl monoglyceridyl citrate or citric acid luminescent molecules.

9. A self-adhesive material prepared by the method for preparing a self-adhesive material according to any one of claims 1 to 8.

10. The self-adhesive material according to claim 9, for use in the following aspects:
the use of preparing a suture-free and/or an anti-adhesion biomembrane; the use of the suture-free and/or the anti-adhesion biomembrane as a tissue anti-adhesion membrane for tendons, abdominal cavity, pelvic cavity, nerves and spinal site.
